# EUROPEAN PATENT SPECIFICATION

(11) **EP 1 919 408 B1**
(45) Date of publication and mention of the grant of the patent: **14.09.2016**
(21) Application number: 06785572.6
(22) Date of filing: 26.06.2006
(51) Int. Cl.: A61F 2/966

(54) **DEPLOYMENT SYSTEM FOR AN EXPANDABLE MEDICAL DEVICE**
ABLAGESYSTEM FÜR EIN EXPANDIERBARES MEDIZINPRODUKT
SYSTÈME DE DÉPLOIEMENT POUR UN DISPOSITIF MÉDICAL EXPANSIBLE

(30) Priority: 30.08.2005 US 216612
(43) Date of publication of application: 14.05.2008
(60) Divisional application: 12170089.2
(73) Proprietor: W.L. Gore & Associates, Inc., Newark DE 19714 (US)
(72) Inventor: CULLY, Edward, H., Flagstaff, AZ 86004 (US); FLURY, Keith, M., Flagstaff, AZ 86004 (US); VONESH, Michael, J., Flagstaff, AZ 86004 (US); ARMSTRONG, Joseph, R., Flagstaff, AZ 86001 (US)
(74) Representative: Wilson, Gary
(86) International application number: PCT/US2006/024782
(87) International publication number: WO 2007/027284

(56) References cited:
- WO-A-00/02503
- WO-A2-2004/066809
- US-A1- 2004 143 272

## Description

### FIELD OF THE INVENTION

The present invention relates generally to implantable medical device assemblies. In particular, the invention relates to means for deploying an expandable medical device within vascular, cardiac or other biologic structures of an implant recipient.

### BACKGROUND OF THE INVENTION

Various implantable medical devices for repairing or reinforcing cardiac, vascular, or other biologic (e.g. biliary tract) structures have been developed in recent years. Some of these devices can be implanted inside a particular vascular or cardiac structure through so-called interventional, or endovascular, techniques. Interventional techniques involve surgically accessing the vascular system through a conveniently located artery or vein and introducing distal portions of a medical device assembly into the vascular system through the arterial or venous access point. Once the medical device assembly is introduced into the vascular system, it is threaded through the vasculature to an implantation site while proximal portions of the assembly having manually operated control means remain outside the body of the implant recipient. The medical device component of the assembly is then deposited at the implantation site and the remainder of the distal portion of the medical device assembly removed from the vascular system through the access point.

Exemplary interventional medical device assemblies include a catheter. The catheter can be used to precisely position the medical device at an implantation site as well as participate in deployment of the medical device at the implantation site. Some catheters have guidewires running their length to aid in positioning and deployment of the medical device. As an alternative to the guidewire, a catheter may be coaxial with an inner sleeve running inside the length of the catheter. The inner sleeve is used to hold an implantable medical device in position while the outer catheter is pulled back causing deployment of the device. Handles, knobs, or other manually operated control means are attached to the opposite end of the catheter in this type of assembly.

Some implantable medical devices, such as stents, stent-grafts, or other endoluminal devices often require reconfiguration from an initial compacted form to an expanded cylindrical configuration as the devices are deployed at an implantation site. These devices can expand on their own by virtue of the design and composition of their structural elements or through the use of an inflatable balloon placed inside the devices.

Self-expanding endoluminal medical devices are maintained in a compacted configuration in a variety of ways. Some devices are maintained in a compacted configuration by simply confining the compacted devices inside a catheter, or similar tool. Other devices are placed inside a sheath following compaction. In these assemblies, a control line is often used to assist in releasing the endoluminal device from the sheath.

In U.S. Patent No. 6,352,561, issued to Leopold et al., a sheath is formed around an expandable endoluminal device and a control line used to maintain the sheath around the endoluminal device. The sheath is formed by folding a length of polymeric material in half and stitching the opposing edges together with the control line. The stitching pattern permits the control line to be removed from the sheath by pulling on a proximal end of the control line. As the control line becomes unstitched from the sheath, the endoluminal device is progressively released from confinement within the sheath. The control line is removed from the assembly as a distinct entity while the sheath remains at the implantation site.

In U.S. Patent No. 5.647.857, issued to Anderson et al., an endoluminal device is held in a collapsed configuration over a catheter by a sheath. The assembly is provided with a control line having a free end and an end attached to a collar component of the catheter. The sheath is removed from the endoluminal device by pulling on the control line. As the control line is pulled, it cuts through and splits the sheath material from distal end to proximal end. As the sheath splits open, the endoluminal device is freed to radially expand. Unlike Leopold et al., the control line remains mechanically attached to the sheath and catheter assembly following deployment of the endoluminal device.

In U.S. Patent No. 6,447,540, issued to Fontaine et al., a confining sheath is removed from around an endoluminal device with a control line that cuts through and splits the sheath material when pulled by a practitioner, much like Anderson et al. As with Leopold et al, the control line can be completely removed from the assembly as a distinct entity.

In U.S. Patent No. 5,534,007, issued to St. Germain et al., a single-walled sheath that can collapse and shorten along its length is placed around a stent. As the distal portion of the sheath is retracted, it uncovers the stent. The uncovered stent is free to expand. An attached control line can be used to exert a pulling force on the collapsible sheath as a means of removing the sheath from the stent. The control line remains attached to the sheath during and subsequent to deployment of the stent.

In U.S. Patent No. 6,059,813, issued to Vrba et al, a double-walled confinement sheath for an endoluminal device is described. In an assembly made of these components, the endoluminal device is placed over a catheter shaft in a collapsed configuration. An outer tube is placed in slidable relationship over the catheter. The distal end of the outer tube does not extend to cover the endoluminal device. Rather, the double walled sheath is placed over the collapsed endoluminal device. The inner wall of the sheath is attached to the catheter shaft near the proximal end of the endoluminal device. The outer wall of the double-walled sheath is mechanically attached to the outer tube. Movement of the outer tube relative to the catheter causes the outer wall of the sheath to move past the inner wall of the sheath. Movement of the outer tube in the proximal direction causes the-sheath to retract and uncover the underlying endoluminal device. As the sheath retracts, the endoluminal device becomes free to expand. A control line is mechanically attached to the outer tube and serves to move the outer tube and retract the sheath.

None of these medical device assemblies utilize a control line that is integral with a sheath. Nor do these assemblies feature a sheath that is convertible to a control line as the sheath is removed from around an expandable medical device, such as an endoluminal device. Such an integral control line and confining sheath would preferably be made of a continuous thin-walled material or composite thereof. The thin-walled material would be flexible and exert minimal restrictions on the flexibility of an underlying expandable medical device. Thin-walled materials would also reduce the profile of the sheath and expandable medical device combination. An integral control line and confining sheath would simplify manufacture of control line-sheath constructs by eliminating the need to mechanically attach the control line to the sheath. An integral control line and confining sheath would also eliminate concerns regarding the reliability of the mechanical attachment of the control line to the sheath. Additionally, inclusion of materials, composites, constructions, and/or assemblies exhibiting compliance, compressibility, resilience, and/or expandability between the sheath-constrained expandable medical device and the delivery catheter would serve to cushion and retain the expandable medical device on a delivery catheter as well as assist in expansion of the expandable medical device in some embodiments.

There is a need, for a reliable deployment system which accurately deploys an expandable medical device as a constraining sheath is gradually removed from the expandable medical device.

US 2004/0,143,272 describes embodiments of deployment systems for an endoluminal device with a constraining sheath, a deployment line and an endoprosthesis mounting member. Embodiments are disclosed where the deployment line is integral with the sheath to aid effectuate device deployment.

### SUMMARY OF THE INVENTION

The present invention is directed to a deployment system for an expandable medical device in the form of an endoluminal medical device as in claim 1 comprising:
a catheter having a length, an outer wall and at least one lumen;
a longitudinal opening in said outer wall that accesses said at least one lumen;
a retractable continuous thin-walled sheath at least partially enclosing the catheter; and
a deployment line configured to pull with the retractable sheath;
wherein said deployment line is integral with the sheath extending through said longitudinal opening, and into said lumen, so that rotation of the catheter relative to the deployment line is prevented.

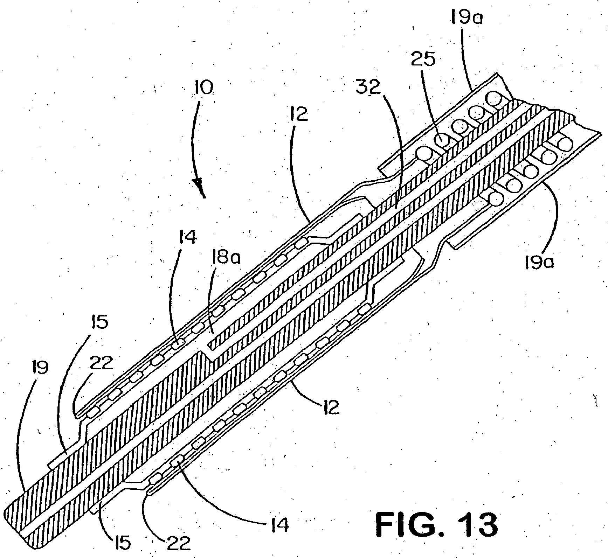

In preferred embodiments, the expandable medical device is expandable with an "endoprosthesis mounting member" or other dilation means placed within the device. In yet other embodiments, the expandable medical device is an inflatable balloon. The expandable medical device is maintained in a compacted, or collapsed, configuration by a removable constraint, in the form of the retractable sheath. In preferred embodiments, the sheath is removed from around the expandable medical device by applying tension to the deployment line attached to or incorporated into the constraint. In the most preferred embodiment, the deployment line is an integral, continuous, extension of a constraining sheath and is made of the same material as the sheath. As the deployment line is pulled, the sheath is progressively removed from around the expandable medical device. When the sheath has been removed from around a portion of the expandable medical device, that portion of the expandable medical device is freed and can be expanded by an underlying endoprosthesis mounting member. Removal of the sheath is continued until the entire expandable medical device is freed from any radial constraint and self-expanded or expanded by the endoprosthesis mounting member. The deployment line along with any remaining sheath material and the endoprosthesis mounting member are removed from the implantation site through the catheter used to deliver the sheathed expandable medical device and underlying endoprosthesis mounting member to the site. In embodiments employing an expandable medical device in the form of a stent, the sheath may be removed from around the stent by inflating an endoprosthesis mounting member, or other dilation means - preferably a balloon. The sheath is removed with the aid of the deployment line portion of the present invention and/or a mechanism capable of storing and releasing kinetic energy. As seen in Figure 13, the mechanism is referred to herein as an "active elastic element (25)"and is preferably in the form of spring elements incorporated into the deployment line portion and/or the sheath portion of the present invention. Alternatively, active elastic elements can be in the form of rubber bands and elastomeric polymers, including fluoroelastomers.

The removable sheath is made of one or more thin, flexible polymeric materials including composites thereof. The sheath ordinarily assumes the form of a continuous thin-walled tube when constraining an expandable medical device, such as an endoluminal device.

The thin-walled sheath exerts minimal resistance to longitudinal flexing of the underlying expandable medical device. The thin-walled sheath also reduces the profile of the sheath - expandable medical device combination, when compared to conventional constraints. In preferred embodiments, a double-walled tubular sheath is used. Double walls enable the sheath to be retracted from around an expandable medical device by sliding one wall past the other wall. As the sheath is retracted, or unrolled, in this manner, the sheath portion does not rub or scrape against the underlying expandable medical device. This is particularly advantageous when coatings containing lubricants, medications, and/or pharmaceuticals are placed on surfaces of the expandable medical device that could be disrupted by a sheath that rubs or scrapes against the expandable medical device as the sheath is removed from the device.

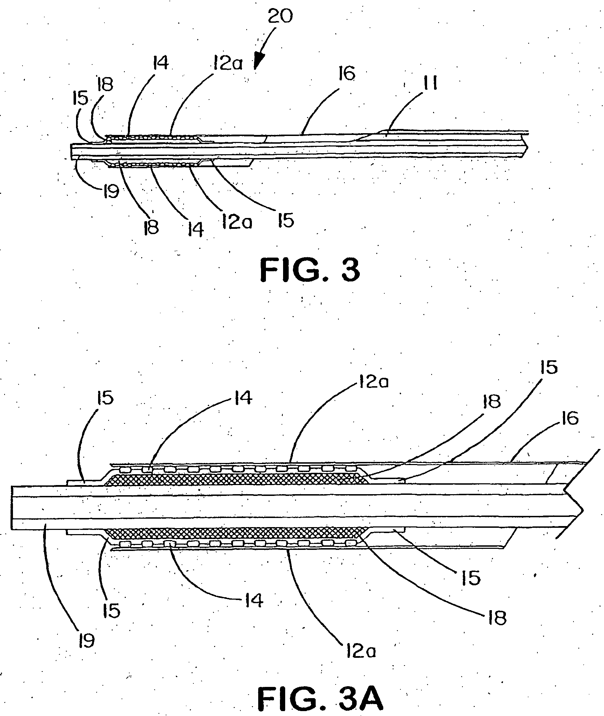

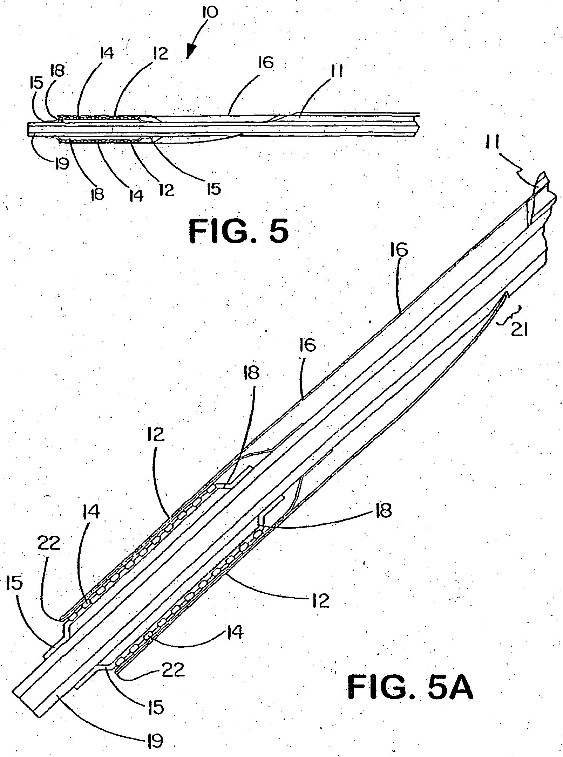

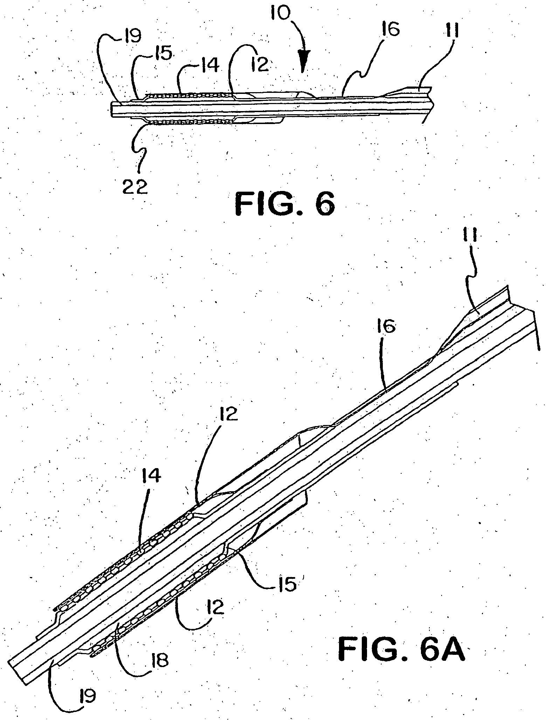

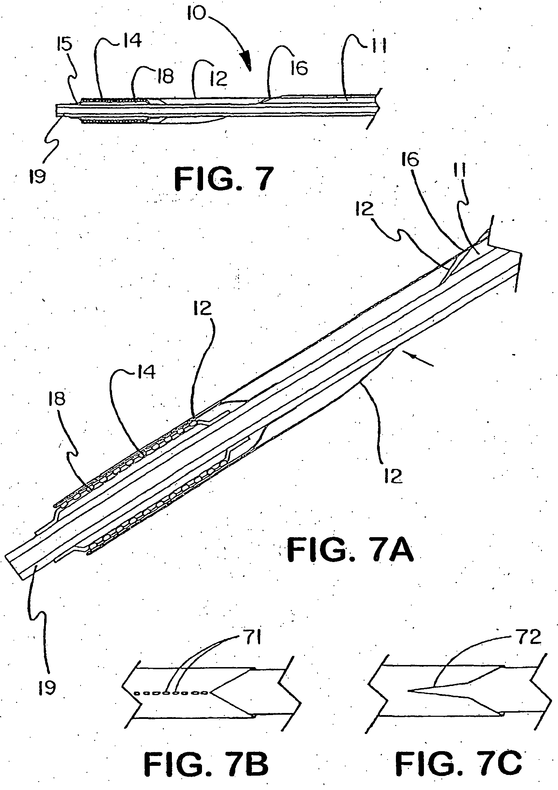

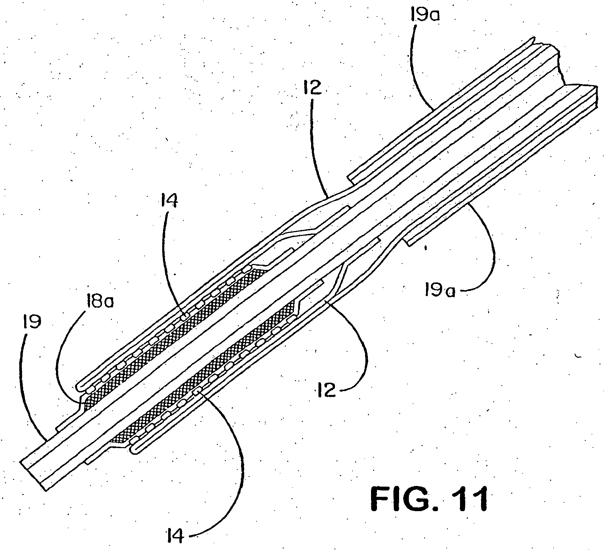

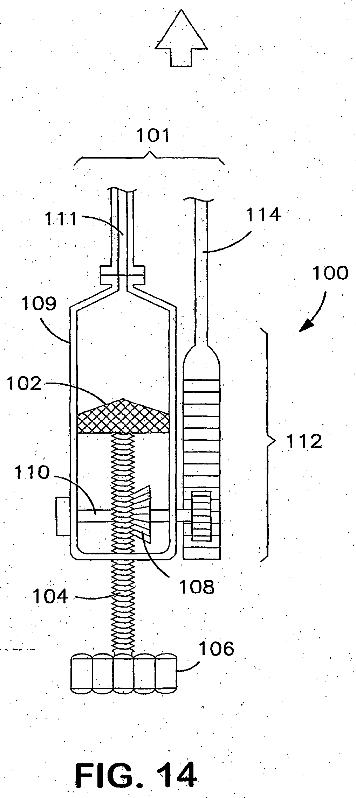

The deployment line is formed from the same material as the removable sheath and is an integral extension of the sheath material. In some embodiments, the deployment line portion (16) extends from the sheath portion (12,12a) through a delivery catheter (19) to a deployment assembly (Figures 14 - 17) located at the proximal end of the catheter (Figures 3 - 7). Among these embodiments, the sheath portion extends proximally beyond the expandable medical device toward the distal end of the deployment system (Figure 5). In preferred embodiments, the sheath extends over the underlying delivery catheter a desired length to a point at which the sheath portion transforms to the deployment line portion (Figure 7). In more preferred embodiments, the sheath portion extends substantially the entire length of the delivery catheter before transforming into deployment line. In the most preferred embodiment (Figure 11), at least a portion of the sheath - deployment line construction (12) is enclosed with a secondary catheter (19a) or catheter lumen, or other containment device such as an expanded porous polytetrafluoroethylene tube. A deployment assembly is provided that simultaneously expands an endoprosthesis mounting member while actuating the deployment line. Once the deployment line is actuated, the removable sheath begins to move, or retract, from around the expandable medical device.

In one embodiment, as removed sheath material travels beyond the receding end of the sheath, the sheath begins to become converted to deployment line. Conversion of the sheath into the deployment line usually begins at a point where the tubular sheath breaks apart, separates, and converges into deployment line material. In preferred embodiments, means are provided for initiating or sustaining the conversion of the sheath to deployment line. These means may take the form of perforations, stress risers, or other mechanical weaknesses introduced into the sheath material. The means can also be cutting edges or sharp surfaces on the delivery catheter.

In preferred embodiments, materials, composites, constructions, and/or assemblies exhibiting compliance, compressibility, resilience, and/or expandability are placed between the endoluminal device and the delivery catheter to provide an "endoprosthesis mounting member." An endoprosthesis mounting member serves to cushion the expandable medical device when constrained by the sheath and may assist in expansion of the device when unconstrained. An endoprosthesis mounting member also serves to anchor and retain the expandable medical, device in place around an underlying catheter shaft. Anchoring the expandable medical device with an endoprosthesis mounting member eliminates the need for barrier, or retention, means at either end of the expandable medical device. The absence of barrier means contributes to a reduction in the profile of the deployment system as well as increasing the flexibility of the distal portion of the system. There can also be provided an additional catheter or catheter lumen for the sheath - deployment line in order to prevent the deployment line portion from leaving the general path established by the delivery catheter. The preferred endoprosthesis mounting member is in the form of an inflatable, or otherwise expandable, balloon. The present invention can also be used alone or in combination with other expandable medical device delivery means. Multiple expandable medical devices can also be delivered with the present invention.

The deployment system uses the endoprosthesis mounting member component of a catheter-based delivery system to exert radial force on an overlying expandable medical device, while simultaneously retracting a sheath-component of the delivery system from around the underlying expandable medical device. By allowing the expandable medical device to be gradually deployed in this manner, adjustments in the position of the device in a patient's vasculature can be made before final deployment of the device. In addition, the deployment, system is particularly useful with expandable medical devices that do not expand completely or as rapidly as desired. In some embodiments, the deployment assembly includes a contrast medium placed within the endoprosthesis mounting member to provide a background against which an expandable medical device can be better imaged.

Accordingly, one embodiment is an implantation system for a medical device comprising an endoprosthesis mounting member, an expandable medical device placed over said endoprosthesis mounting member, a constraint placed over at least a portion of said expandable medical device, and a delivery catheter incorporating said endoprosthesis mounting member, constraint, and expandable medical device, wherein said expandable medical device is deployed by simultaneous retraction of said constraint from said expandable medical device and expansion of said endoprosthesis mounting member.

In another embodiment, there is a medical device deployment system comprising a catheter tube having a proximal end, a length, and a distal end, an endoprosthesis mounting member placed on said distal end of said catheter tube, and an expandable medical device placed over said endoprosthesis mounting member and covered with an overlying retractable sheath, said sheath incorporating a deployment line running inside said catheter tube and attached to an actuator at said proximal end of said catheter tube, wherein said actuator is coupled to a means in fluid communication with a lumen of said endoprosthesis mounting member for expanding said endoprosthesis mounting member, whereby said endoprosthesis mounting member is expanded simultaneously with retraction of said overlying sheath from said expandable medical device.

In yet another embodiment, there is a deployment assembly for a catheter-based delivery system comprising a container defining a first pressurizable chamber, said first pressurizable chamber having an opening therein configured to attach to a delivery catheter having an endoprosthesis mounting member incorporated thereon and provide fluid communication between said first pressurizable chamber and a lumen of said endoprosthesis mounting member, a movable plunger placed within said first pressurizable chamber to establish and maintain fluid pressure in said endoprosthesis mounting member lumen when said plunger is moved, and a deployment line actuator coupled to said movable plunger.

In yet another embodiment, for sheath pull back catheters that include a deployment line transitioning into a coaxial sheath, the present invention encapsulates the majority of the deployment line length within the catheter extrusion. The deployment accuracy of the system is improved by utilizing a longitudinal slit or groove in the catheter for insertion of the deployment line completely into one of the lumens of the catheter. The slit allows the junction of the deployment line and the sheath to travel along a length of the catheter when the sheath is retracted. An advantage of the deployment line being located inside the catheter at locations proximal of the deployment line/sheath junction, is that the outside diameter of the proximal end of the catheter remains completely stationary during deployment.

In yet another embodiment, the retractable sheaths can be constructed from high density ePTFE membranes to reduce the deployment force because of their high density smooth, lubricous contact surface.

These and other embodiments can also include a pressure relief valve in fluid communication with a pressurizing chamber to prevent over pressurization of an endoprosthesis mounting member. Enclosed gas bladders, or other "pressure capacitors," can also be placed in a pressurizing chamber to help maintain pressurization of an endoprosthesis mounting member.

These enhanced features and other attributes of the deployment system of the present invention are better understood through review of the following specification.

### BRIEF DESCRIPTION OF THE DRAWINGS

Figure 1 illustrates a longitudinal cross-section of the present invention.
Figure 1 A is an enlarged view of Figure 1.
Figure 2 illustrates a perspective view of the present invention.
Figure 3 illustrates a longitudinal cross-section of the present invention.
Figure 3A is an enlarged view of Figure 3.
Figure 4 illustrates a longitudinal cross-section of the present invention.
Figure 4A is an enlarged view of Figure 4.
Figure 5 illustrates a longitudinal cross-section of the present invention.
Figure 5A is an enlarged view of Figure 5.
Figure 6 illustrates a longitudinal cross-section of the present invention.
Figure 6A is an enlarged view of Figure 6.
Figure 7 illustrates a longitudinal cross-section of the present invention.
Figure 7A is an enlarged view of Figure 7.
Figure 7B illustrates the embodiment of Figure 7A as viewed from the direction indicated by the arrow.
Figure 7C illustrates the embodiment of Figure 7A as viewed from the direction indicated by the arrow.
Figures 8 and 8A illustrate longitudinal cross-sections views of the present invention placed inside a vascular or cardiac structure.
Figure 9 illustrates a longitudinal cross-section of the present invention with a covering placed over an endoprosthesis mounting member.
Figure 9A illustrates a longitudinal cross-section of the present invention without a covering placed over an endoprosthesis mounting member.
Figure 10 illustrates a longitudinal cross-section of the present invention with an endoprosthesis mounting member placed between an underlying delivery catheter and an expandable medical device.
Figure 10A illustrates the longitudinal cross-section of Figure 10 with the endoprosthesis mounting member in a partially expanded configuration, the sheath in a partially retracted configuration, and the expandable medical device in a partially expanded configuration.
Figure 11 illustrates a longitudinal cross-section of the present invention having an outer catheter, or tube, placed over substantially the entire length of a sheath - deployment line construction.
Figure 12 illustrates a longitudinal cross-section of the present invention showing an expandable medical device in the form of a collapsed and folded inflatable balloon having a first dimension confined to a second dimension with a sheath - deployment line of the invention.
Figure 13 illustrates a cross-section of the present invention showing an active elastic element attached to the sheath portion of the present invention as a means to remove the sheath from around an expandable medical device.
Figure 14 illustrates a deployment assembly of the present invention having a chamber in fluid communication with a catheter lumen and a plunger in the chamber for pressurizing and inflating an endoprosthesis mounting member. The plunger is coupled to a rack and pinion gear combination through an axle and bevel gear. In practice, actuation of the plunger simultaneously pressurizes and inflates an endoprosthesis mounting member while actuating the pinion gear to move the rack and retract a deployment line or rod attached to, or incorporated into, a sheath component of the deployment system.
Figure 15 illustrates a deployment assembly of the present invention showing a chamber in fluid communication with a catheter lumen and a plunger in the chamber for pressurizing and inflating an endoprosthesis mounting member. The plunger is coupled to a spool, reel, or drum, through an axle and bevel gear. In practice, actuation of the plunger simultaneously pressurizes and inflates an endoprosthesis mounting member and rotates the spool to retract a deployment line or rod attached to, or incorporated into, a sheath component of the deployment system.
Figure 16 illustrates a deployment assembly of the present invention showing a chamber in fluid communication with a catheter lumen and a plunger in the chamber for pressurizing and inflating an endoprosthesis mounting member. The plunger is coupled to a deployment line, rod, or retractable sheath through a pulley. In practice, actuation of the plunger simultaneously pressurizes and inflates an inflatable balloon and moves the deployment line, rod, or retractable sheath through the pulley to retract the deployment line, rod, or retractable sheath component of the deployment system.

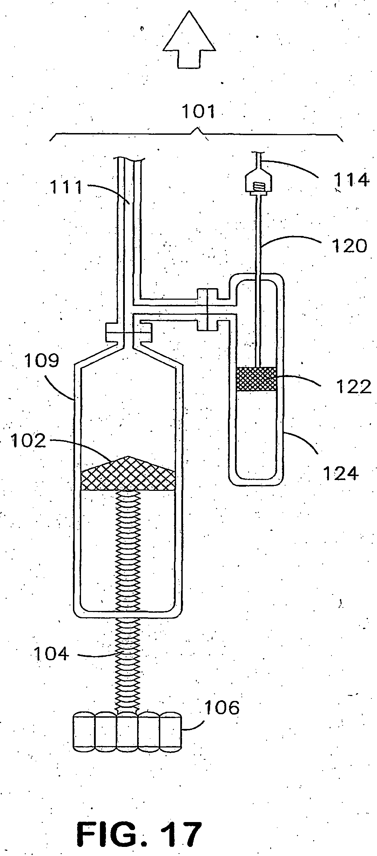

Figure 17 illustrates a deployment assembly of the present invention showing a first chamber in fluid communication with a catheter lumen and a plunger in the chamber for pressurizing and inflating an inflatable endoprosthesis mounting member. A second chamber is attached to the assembly in fluid communication with the first chamber and catheter lumen. A piston is placed in the second chamber and is attached to a deployment line, rod, or retractable sheath component of the deployment system. In practice, actuation of the plunger simultaneously pressurizes and inflates an inflatable endoprosthesis mounting member while moving the piston to retract the deployment line, rod, or retractable sheath component of the deployment system.
Figure 18 illustrates a pressure storage component of the present invention.
Figure 19 illustrates a pressure storage component of the present invention.
Figure 20 illustrates a pressure storage component of the present invention.
Figures 21 A-C illustrate a sheath pull back catheter of the present invention with a longitudinal opening prior to retraction of the sheath.
Figures 22 A-B illustrate a sheath pull back catheter of the present invention with a longitudinal opening post-retraction of the sheath.
Figure 23 A- B illustrate the relative non-rotative movement in the present invention upon deployment of an endoluminal device.
Figure 24 illustrates the initial force required to transition from a static state to a dynamic state is materials dependant.

### DETAILED DESCRIPTION OF THE INVENTION

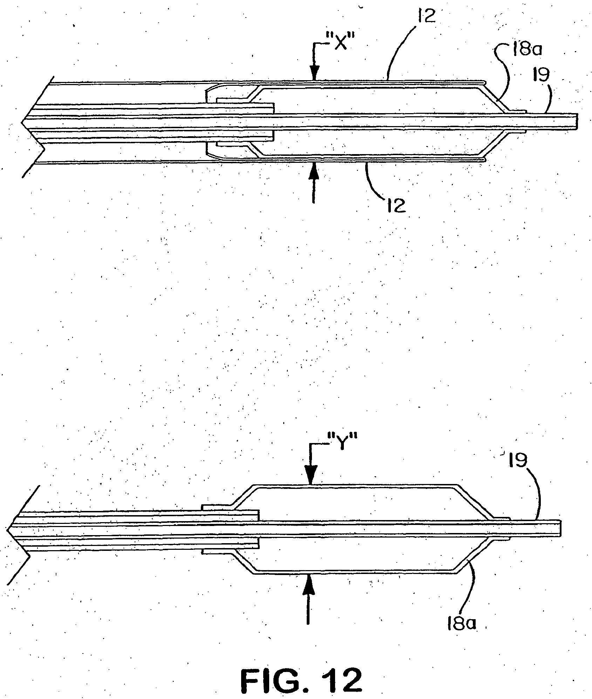

There is a deployment system for an expandable medical device, such as an endoluminal device, having a removable sheath with a deployment line or filament that is an integral part of the sheath. As indicated by the relative difference in the space between the "x" arrows and the "y" arrows in Figure 12, the sheath portion (12) confines the endoluminal device (18a) to a smaller profile than is possible without the sheath. The sheath radially confines, or constrains, the expandable medical device in a compacted or collapsed configuration during storage and introduction into a patient's vascular system. The constraining sheath maintains the expandable medical device in a compacted configuration until the device is delivered with a catheter to an implantation site in a vascular or cardiac structure. At the time of deployment, the sheath is retracted from the expandable medical device. In some embodiments, sheath material may be converted into deployment line material as the sheath is removed from the expandable medical device. As the sheath is removed from the expandable medical device, the expandable medical device is freed to expand. Once freed from the confining sheath, the expandable medical device may expand spontaneously or with the assistance of an endoprosthesis mounting member. Any remaining sheath material may be removed from the implantation site along with the deployment line.

The deployment system of the present invention permits retraction of the constraining sheath simultaneously with expansion of an endoprosthesis mounting member. The present invention is also directed to a deployment assembly that accomplishes simultaneous expansion of an endoprosthesis mounting member and removal of a constraining sheath from an expandable medical device.

The integral sheath - deployment line is preferably a flexible polymeric material that is continuous along the length of the construct. Preferably, the physical and mechanical properties of the sheath portion are such that they are uniform and homogeneous throughout the length of the sheath portion used to constrain the expandable medical device. Since most expandable medical devices are generally circularly cylindrical in form, the sheath is preferably tubular in shape in order to enclose most or all of the expandable medical device. Conical, tapered, or other suitable shapes for the sheath are also contemplated in the present invention. Flexibility of the sheath is enhanced by making the walls of the sheath as thin as practicable. In one embodiment of the present invention (20), the tubular sheath portion (12a) of the sheath - deployment line has a single wall (Fig. 3). The deployment line portion can extend from either end of the single-walled sheath (12a). When the sheath portion is retracted from around an expandable medical device, the length of retracted sheath is substantially equal to the length of deployment line displaced during deployment of the expandable medical device.

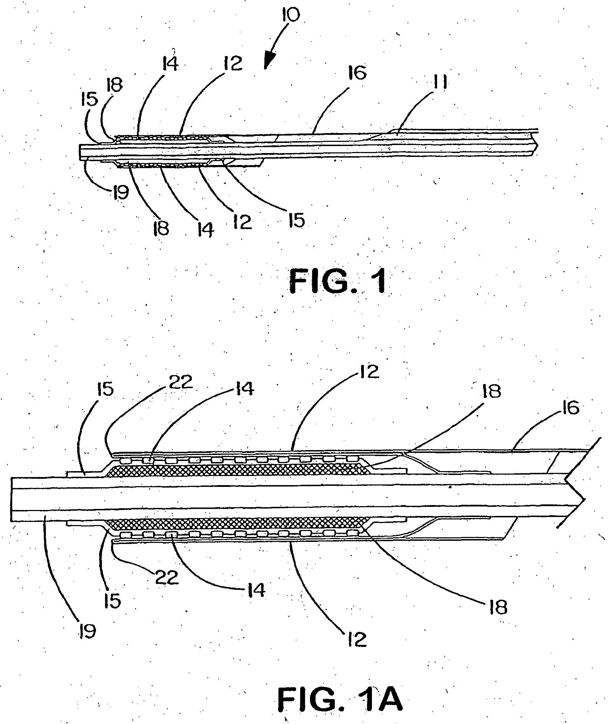

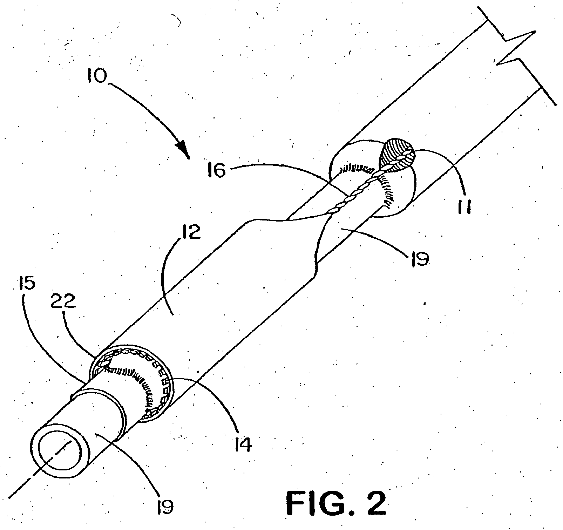

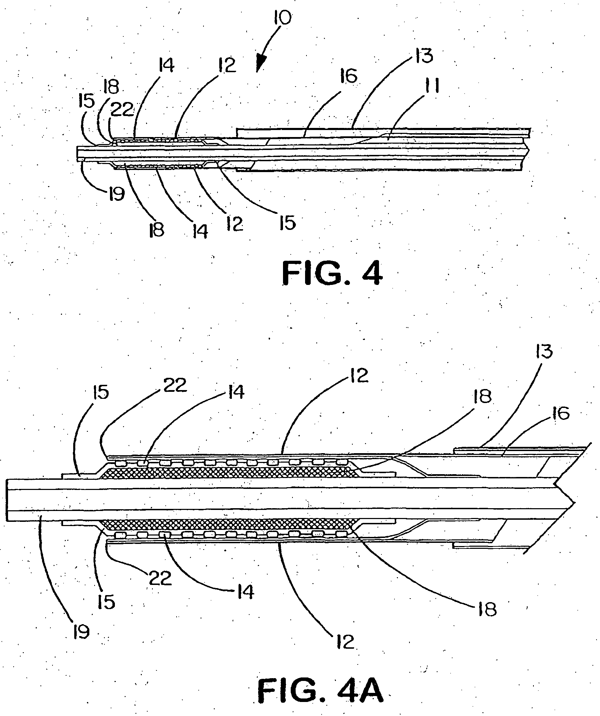

In another embodiment of the present invention (10), the sheath portion (12) of the sheath - deployment line has a double wall (Figs. 1, 2, and 4 - 11). In a preferred embodiment, the double walled-sheath portion (12) is made of a polymeric material that is folded on itself. The double-walled sheath portion is placed over the expandable medical device (14) so that the fold (22) is positioned at the distal end (*i.e*., farthest from the deployment assembly) of the sheath portion (12). The inner wall of the sheath portion may be anchored to part of an underlying delivery catheter (19) proximal to the expandable medical device (14). In preferred embodiments, the sheath portion (12) is not attached to the delivery catheter (19). The proximal end of the outer wall of the sheath has at least one portion, or integral extension, that is convertible to deployment line (16). Space between the walls of the double-walled sheath portion can be filled with fluids, lubricants, pharmaceutical compositions, and/or combinations thereof. The deployment line (16) is routed through the delivery catheter (19) to a deployment assembly of the present invention (e.g. Figures 14 - 17) located at the proximal end of the deployment system (10). Alternatively, a separate catheter (13) or catheter lumen (11) is provided for the deployment line (Figs 4 and 1, respectively). These embodiments provide additional containment of the deployment line portion, particularly when bends or curves in a patient's vasculature having small radii are anticipated. In the most preferred embodiment (Fig. 11), the sheath portion of the sheath - deployment line construction extends substantially the entire length of the delivery catheter (19) and is confined within a separate catheter (19a) or catheter lumen. The deployment line portion is formed near the proximal end of the deployment system and is attached to a deployment assembly (e.g. Figures 14 -17).

Preferably, the physical and mechanical properties of the sheath portion are such that they are uniform and homogeneous throughout the length of the sheath portion used to constrain the expandable medical device. When the sheath portion is retracted from around an expandable medical device, the length of retracted sheath is essentially half the length of deployment line displaced during deployment of the expandable medical device. This two to one ratio (2:1) of length of deployment line removed to length of sheath material removed reduces the effect of too rapid or strong a pull on the deployment line on release of the expandable medical device from the sheath.

Fluoropolymer materials are preferred for making the retractable tubular constraining sheath - deployment line constructs of the present invention. Fluoropolymer materials used in the present invention are strong, thin, and lubricious. The lubriciousness of the fluoropolymer materials is especially advantageous in embodiments utilizing a sheath -deployment line having walls that slide past one another or over an expandable medical device. Particularly preferred fluoropolymer materials are porous expanded polytetrafluoroethylene materials alone or in combination with fluorinated ethylene propylene materials. Most preferred fluoropolymer materials are strong and thin, such as those described in Example 2, *infra.* The sheath - deployment line is made by constructing an appropriate tube from layers of film and/or membrane. The sheath-deployment line may also be constructed from extrusions of polymeric materials. The extrusions can be used alone or in combination with film/membrane materials. Once constructed, a significant portion of the tube is rendered filamentous by rolling and heating.

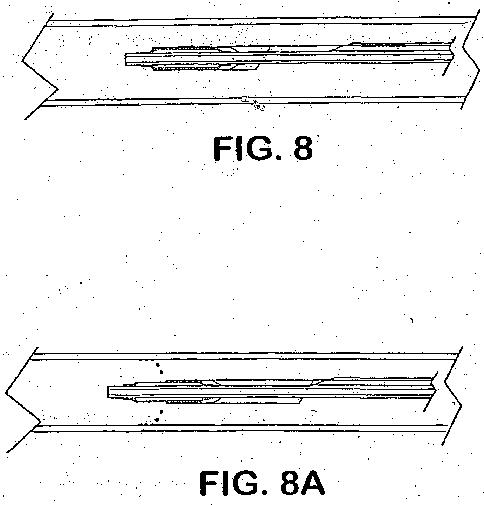

The sheath may be converted to deployment line by pulling on the deployment line and causing the sheath material to separate and converge into a single filament. As sheath material is converted to deployment line by this process, the edge of the sheath supplying material to the deployment line recedes causing the sheath to retract from around the expandable medical device. As a portion of the sheath retracts, the portion of the expandable medical device confined by the sheath is freed to expand (Figs 8 - 8A). Means are optionally provided to the deployment system that initiate or sustain the conversion of sheath to deployment line. As shown in Figure 7, the means include perforations (71), cutouts (72), or other engineered defect introduced into the sheath material. As shown in Figure 5, the means also include cutters (21) or other sharp edges on the delivery catheter. Such cutting means may be formed on the delivery catheter by exposing a strand of reinforcing stainless steel from within the catheter and adapting the strand to cut into the sheath portion.

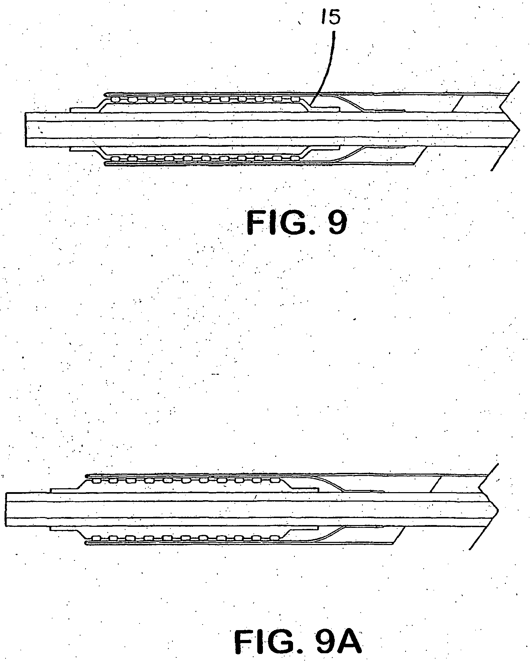

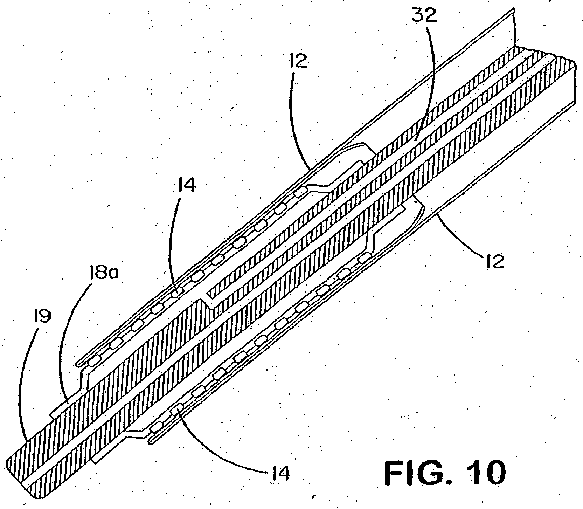

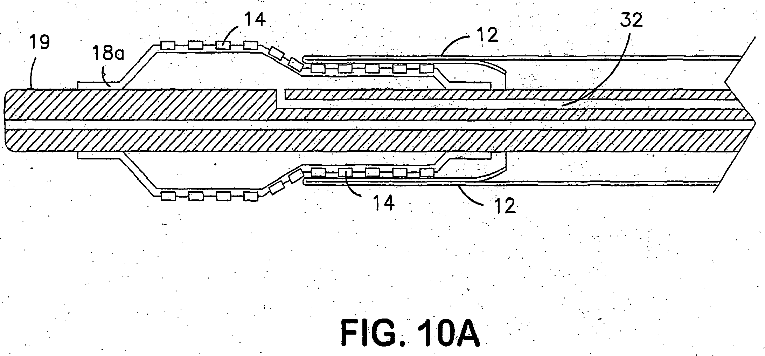

In the preferred embodiment of the present invention, materials, composites, constructions, and/or assemblies exhibiting compliance, compressibility, resilience, and/or expandability are placed between the expandable medical device and the delivery catheter to form an "endoprosthesis mounting member (18)." The endoprosthesis amounting member can be covered (15) or uncovered (Fig. 9). At least a portion of the expendable medical device is pressed into a covered or uncovered endoprosthesis mounting member to anchor the expandable medical device on the delivery catheter and prevent the expandable medical device from moving along the length of the catheter. Materials with a tacky surface are useful with the endoprosthesis mounting member, particularly in combination with a lubricious sheath material. The endoprosthesis mounting member eliminates the need for barrier, or retention, means placed at the proximal and distal end of the expandable medical device. In addition to added flexibility imparted to the deployment system without the barrier means, the profile of the sheath and expandable medical device combination is reduced without the barrier means. In yet another embodiment, the endoprosthesis mounting member is in the form of an inflatable balloon (Fig 10, part 18a). Suitable materials for the endoprosthesis mounting member include, but are not limited to, silicones, silicone foams, polyurethane, polyurethane foams, and polytetrafluoroethylene foams or combinations thereof. The endoprosthesis mounting member is attached to the outer wall of the delivery catheter with adhesives, heat, or other suitable means. An inflatable endoprosthesis mounting member (18a) has at least one lumen in fluid communication with at least one lumen of the delivery catheter tube, which in turn, is in fluid communication with a pressurizable first chamber component (109) of a deployment assembly (100) of the present invention.

A non-inflatable endoprosthesis mounting member is preferably enclosed with a covering (15) in the form of a polymeric material. The polymeric material is preferably a fluoropolymer-based material. Porous expanded polytetrafluoroethylene is the preferred fluoropolymer for enclosing the compressible material. Other suitable polymeric materials include, but are not limited to, silicone, polyurethane, polyester, and the like.

The present invention is also directed to a system for deploying the expandable medical device. Once the above-described expandable medical device deployment system is maneuvered to a desired location in a patient's vasculature, the system is activated to expand the endoprosthesis counting member, while the sheath is simultaneously retracted from the expandable medical device. This step is often performed gradually to expose only portions of the expandable medical device at a time (Figure 10A). The controlled deployment of the expandable medical device offered with the present invention permits the location of the device in the vasculature to be verified and adjusted, if necessary, before final deployment of the device. In some deployments, the sheath component of the system may become difficult to remove from an underlying expandable medical device. The deployment system of the present invention assists in such deployment by providing a mechanical advantage to the deployment line - sheath component that allows sufficient pulling force to be applied to the component to remove the sheath from the expandable medical device.

Preferred endoprosthesis mounting members are inflatable (18a). The inflatable endoprosthesis mounting member has a lumen (32) in fluid communication with a deployment assembly (100) of the present invention. Fluid in the form of gas or liquid is introduced into the endoprosthesis mounting member from a pressurizable first chamber (109, Figures 14 -20) in the deployment assembly (100). As fluid pressure is generated in the first chamber (109), the pressure is transferred to the endoprosthesis mounting member (18a) where the pressure exerts force radially against an overlying expandable medical device (14). As the endoprosthesis mounting member becomes pressurized, an actuator (112, 116, 118, 120/122) coupled to the pressurization means (102/109) simultaneously begins to retract the sheath (12) from the expandable medical device (14). Further retraction of the sheath (12) allows the endoprosthesis mounting member to cause, assist, or permit radial expansion of exposed portions of the expandable medical device. Pressure is maintained in the endoprosthesis mounting member by the deployment assembly as the sheath is completely removed from the expandable medical device and the device fully deployed.

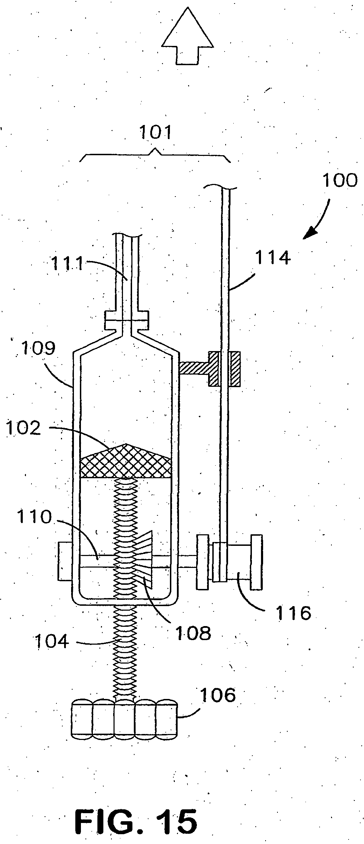

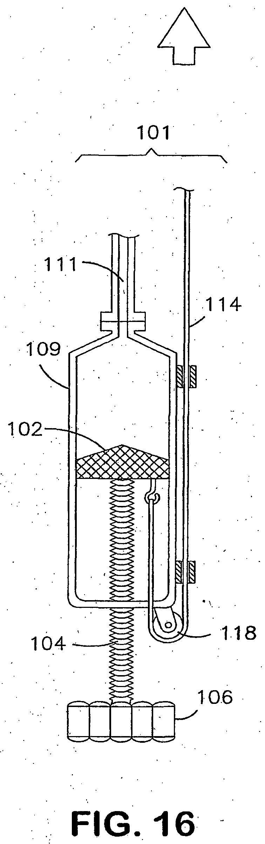

Retraction of the sheath is accomplished simultaneously with expansion of the endoprosthesis mounting member in the present invention by attaching the deployment line portion of the deployment line - sheath combination to an actuator that is mechanically coupled with a plunger, piston, or other fluid pressurization means. As seen in Figures 14 - 20, chamber (109) houses a plunger (102) attached to a first gear (104) or other screw means. The first gear (104) has a knob (106), handle, motor-drive coupler, or other activator for turning the first gear (104). A bevel, or similar, gear (108) engages the first gear (104) and is attached to an axle (110) that in turn in attached to an actuator (112, 116, 118, 120/122) for retracting a sheath from an expandable medical device. In one embodiment (Figure 14), the actuator includes a rack and pinion gear arrangement attached to a deployment line (114). In another embodiment (Figure 15), the actuator includes a deployment line (114) attached to a reel (116) connected to axle (110). In yet another embodiment (Figure 16), the actuator includes deployment line (114) attached to a pulley (118).

In a preferred embodiment (Figure 17), the actuator includes a deployment line (114) attached to a rod (120) connected to a piston (122) inside a second chamber (124) in fluid communication with the first chamber (109). As fluid pressure is increased in the first chamber (109), the pressure increases in both the endoprosthesis mounting member and the second chamber. Increased pressure in the second chamber causes the piston to move and pull on the deployment line. In turn, this causes the sheath to retract from the endoluminal device.

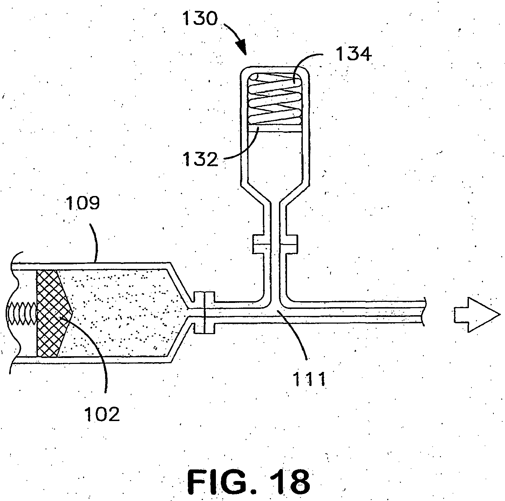

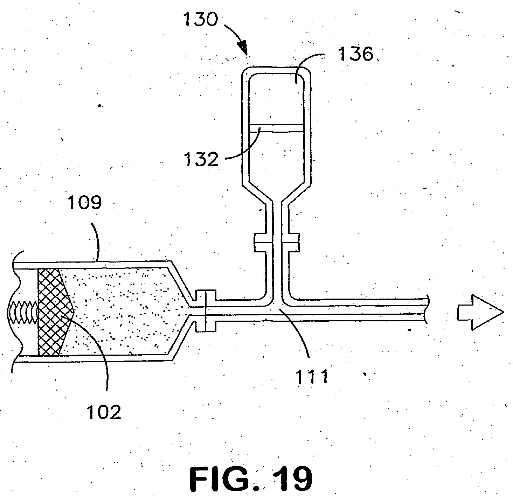

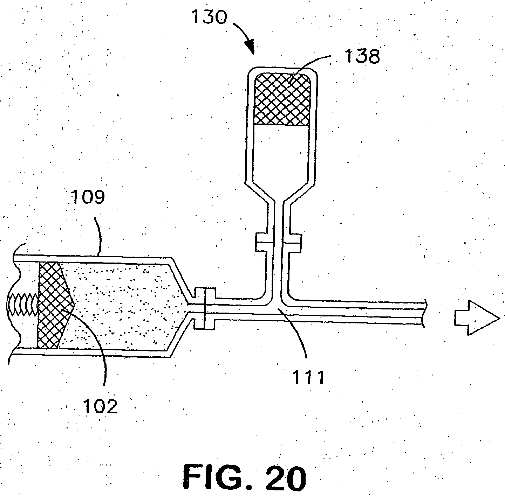

In some situations, it may be desirable to pressurize and begin to expand an endoprosthesis mounting member before activating the deployment line and retracting the sheath. Figures 18 - 20 illustrate examples of pressure-storing apparatuses for use in pressurizing and expanding an endoprosthesis mounting member. Figure 18 shows a third chamber (130) in fluid communication with the first chamber (109) having a diaphragm (132) having a spring (134) exerting mechanical force on the diaphragm (132). Figure 19 shows a third chamber (130) in fluid communication with the first chamber (109) having a diaphragm (132) and a gas (136) exerting mechanical force on the diaphragm (132). Figure 20 shows a third chamber (130) in fluid communication with first chamber (109) having a compressible material (138) in the third chamber (130).

Lastly, a contrast medium can be incorporated or introduced into the endoprosthesis mounting member to better visualize an overlying endoluminal device.

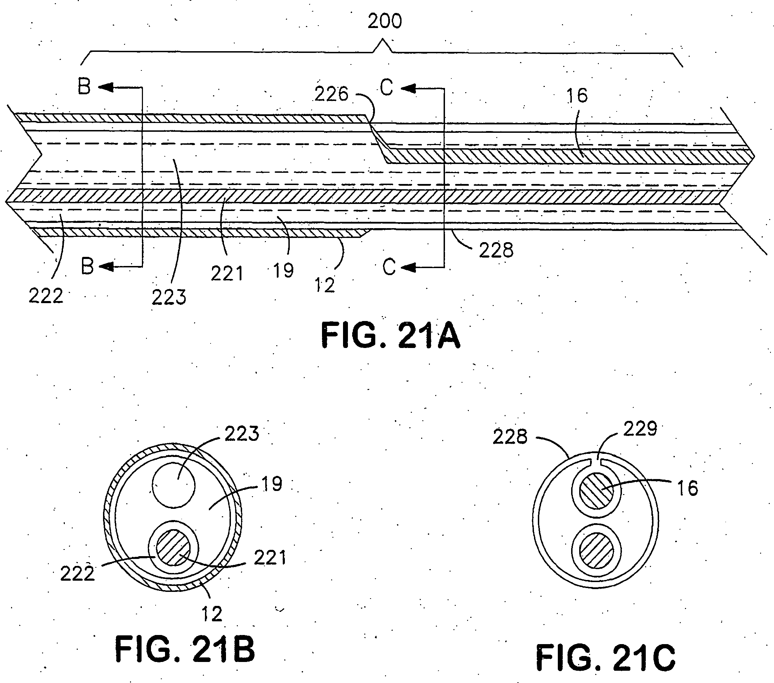

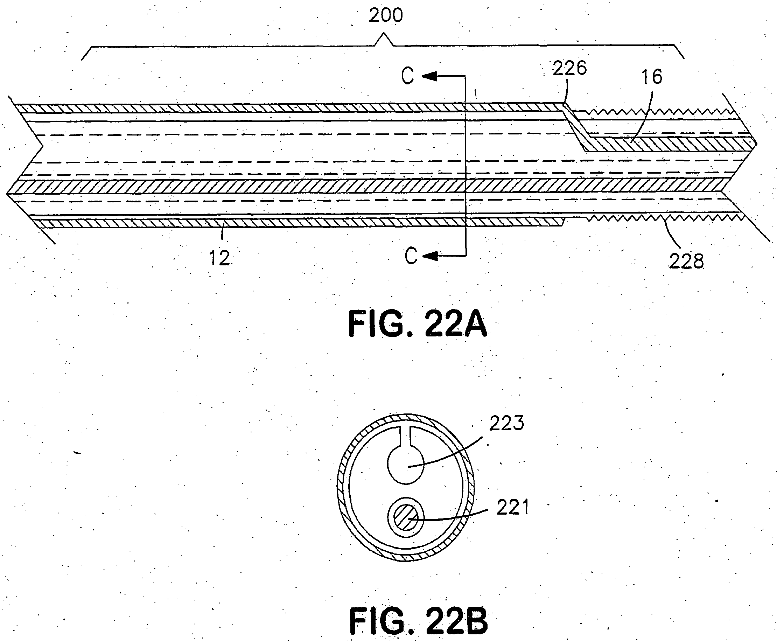

It is further desirable to enhance deployment accuracy. Figures 21A-C and 22 A-B show a sheath pull back catheter embodiment (200) of the present invention. The deployment system of this embodiment comprises a catheter (19), a retractable sheath (12); a deployment line (16) and a deployment line lumen (223). The device comprises a catheter (19) with at least two lumens (222, 223). The retractable sheath (12) may be configured to surround at least a portion of the endoluminal device and constrain the device in an introductory profile. A deployment line (16) is inserted into a longitudinal opening (229) in the catheter, and is configured to pull with the sheath (12). The deployment line (16) may also be configured to pull other individual components. The catheter (19) is positioned around a slideable or fixed guide wire (221) positioned within the extrusion's guide wire lumen (222). The sheath may be used to restrain a device (e.g. a self expanding stent or filter, not shown) or devices on the catheter extrusion for delivery of the device(s) into a patient. By retracting (i.e. applying a pulling force to the deployment line in Figure 21A and 22A) the deployment line (16) connected to the sheath (12), the sheath (12) can be withdrawn from over the device(s) allowing it the expand. This expansion can be caused by elastic recovery, by secondary mechanical means (e.g. a balloon or dialator), by thermal means, by electrical means, or by other means. Figure 21A shows a catheter (19) with two lumens. A longitudinal opening (229) or gap accesses one of the lumens for transitioning a deployment line (16) into a sheath (12). The deployment accuracy of the system is increased by a longitudinal opening (229) in the catheter which allows insertion of the deployment line (16) completely into a deployment line lumen (223) of the catheter. By keeping the majority of the length of the deployment line (16) within this deployment line lumen at all times during device deployment, the catheter extrusion can not twist around the tensioned deployment line. The longitudinal opening (229) in the deployment line lumen allows the junction (226) of the deployment line and the sheath (12) to travel along a length of the catheter when the sheath (12) is retracted. Prior to deployment, the un-tensioned deployment line (16) minimally impacts the delivery flexibility of the catheter. When the deployment line (16) is tensioned for deployment, the line becomes very stiff and as it is incorporated within the catheter, the amount of twisting or shortening is minimized around the deployment line. Thus, deployment reliability and accuracy is greatly improved as compared to traditional deployment devices that incorporate a deployment line.

Figures 21A and 22A show a sheath pull back catheter embodiment of the present invention. The catheter is positioned around a guidewire (221) fed through a guide wire lumen (222). There are two cross sectional regions shown at Figures 21B and 21C which illustrate the present invention prior to sheath retraction. As shown at Figure 21A and 21C a longitudinal opening (229) provides access to a deployment line lumen (223) for transitioning the deployment line (16) into a coaxial sheath. The longitudinal opening allows the junction (226) of the deployment line and the sheath to commence movement along a length of the catheter when the sheath (12) is retracted. The sheath (12) may be formed of any suitable material, and is preferably a continuous film or material. It is advantageous in some instances that the sheaths have a low coefficient of friction, and have a thin wall. Materials that incorporate the attributes of strength, lubricity and thinness include but are not limited to: polymeric materials, polytetrafluoroethylenes, high density polyethylenes, polyimides, nylons, polyamides, and PEEK. The polytetrafluoroethylenes include porous expanded polytetrafluoroethylene, such as ePTFE. Tubular sheaths can be manufactured out of ePTFE films by wrapping different layers in either longitudinal or circumferential directions and sintering them together. A sheath (12) may be created to optimize thin low profiles. In order to create a sheath with a thin profile and a very low coefficient of friction, it has been found that the sheath can be densified further after sintering. This further densification may be induced manually by compressing the outside diameter of the sheath. One method of compression is repeated rubbing or pressing of a polymer mandrel over the sheath using a high normal force. The sheath is preferably loaded onto a mandrel during this densification process. Alternatively, high density ePTFE films can be used as a base film to produce these high density sheaths. Films or materials with a density of greater than 1.5 g/cc are favored, or more preferably films or materials with a density of greater than 2.0 g/cc or, more preferably, greater than 2.1 g/cc. These sheaths are preferably translucent, indicating the sheath is approximately full density and additionally allowing inspection of a stent or other device within the sheath.

The deployment line may be made of any suitable material, such as but not limited to: metals, polymeric materials, polytetrafluoroethylenes, high density polyethylenes, polyimides, nylons, polyamides, PEEK. It is desired that the deployment line be a high strength lubricous material. The deployment line is configured to pull with the second portion of the removable sheath (12). The delivery catheter (19) may comprise a single catheter shaft, or may comprise more than one catheter shaft.

A coaxial cover (228) may be positioned over the longitudinal opening in the catheter. It is preferred that the coaxial cover (228) has a density greater than 1.5 g/cc. The deployment line (16) may be configured with the sheath as a single piece, or multiple joined pieces. When the deployment line and sheath are one single piece it may be formed to have at least two discrete sections. Discrete sections of the piece may be transformed by processes such as drawing a portion of the line through a small diameter heated die, or any other transformation process. As an actuation force is exerted from the deployment line to the sheath, the sheath begins to move and effectuate device deployment. The length of sheath (12) retracted from the device is substantially equal to the length of deployment line (16) displaced during the deployment of a self-expanding endoluminal device.

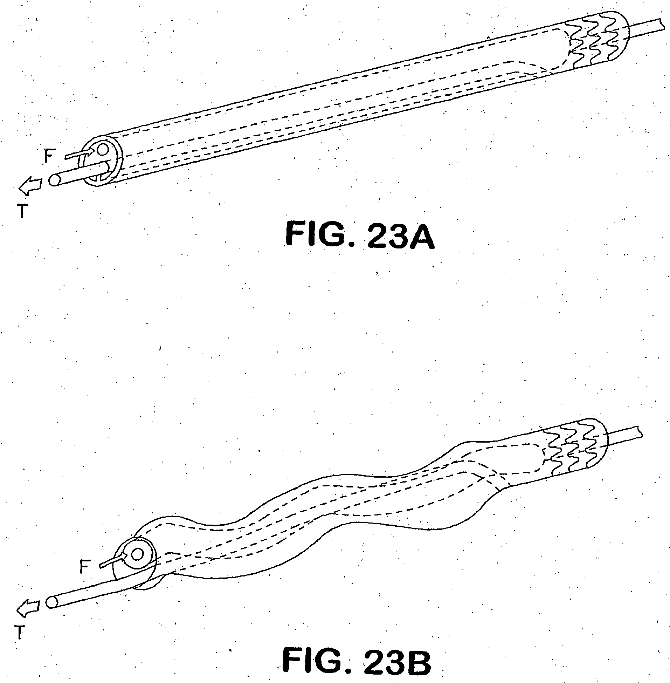

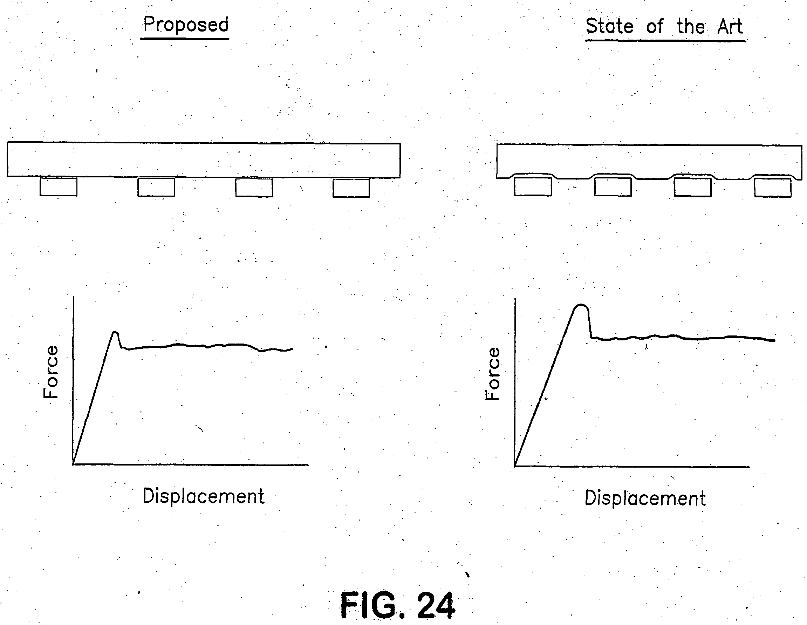

The deployment line lumen (223) may be configured to completely encase the majority of the length of the deployment line (16) and allow deployment line movement. The majority of the length of the deployment line (16) is considered to be greater than half of the deployment line. It is preferred that the encased majority of length of the deployment line (16) is greater than 90% of length of the deployment line. In certain embodiments it is desired that the encased majority of length of the deployment line (16) is 95% of length of the deployment line. In other certain embodiments, it may be desired that the encased majority of length of the deployment line is 99% of length of the deployment line. The deployment line lumen (223) is configured to prevent rotation of the catheter relative to the deployment line during deployment line actuation. Rotation of the catheter is considered to be any movement less than 360 degrees. It is desired that the rotation of the catheter is less than 180 degrees, and preferably, less than 90 degrees. The catheter (19) has an outside diameter proximal of the coaxial cover (228) which remains essentially stationary during deployment. As shown in Figures 23A, as a force (F) is applied to the deployment line situated within the deployment line lumen, the catheter is prevented from rotating relative to the deployment line. Thus, as the retractable sheath (12) is retracted, the endoluminal device is released and shortening of the catheter is minimized. Figure 23B shows that a force applied to a deployment line which does not employ a deployment line lumen as taught by the present invention moves in a rotative manner at the distal end. Thus, In Figure 23B twisting of the catheter occurs at the distal end resulting in additional movement of the endoluminal device during deployment. Figure 24 shows that the initial force required to transition the retractable sheath (12) from a static state to a dynamic state is reduced by choosing materials which allow ease of movement between the endoluminal-device and the retractable sheath material in contact with the endoluminal device. Suitable materials for the retractable sheath material in contact with the endoluminal device include polymers including but not limited to fluoropolymers such as expanded polytetrafluoroethylene, however any suitable material may be used. Reduction of the initial force exerted on the deployment line also aids in the accurate device placement by reducing the compressive force placed on the catheter.

A preferred deployment system for a self-expanding endoluminal device comprises a self-expanding endoluminal device with at least one lumen and at least partially enclosed by a retractable sheath (12); and a deployment line (16) configured to pull the sheath (12). The deployment system for a self-expanding endoluminal device may further comprise means on the catheter for initiating conversion of the sheath (12) to the deployment line (16). The deployment system may be configured to allow the retractable sheath to be split by the means to initiate conversion of the removable sheath to the deployment line.

Figure 21A is shown in cross section at Figure 21C, the deployment lumen (223) and the guidewire lumen (222) are present inside the delivery catheter (19). The deployment lumen in Figure 21B is not occupied by the deployment line as the sheath is circumferentially covering the exterior of the device prior to deployment. In this region the deployment lumen (223) comprises a longitudinal opening (229) here shown in the shape of a slit which allows access to the lumen in which the deployment line (16) is located. As the device is deployed, the sheath transitions into the deployment line (16) and move through the deployment line lumen (223). The guidewire lumen (222) continues through regions in Figures 21B and 21C, as shown.

While the present invention is applicable to numerous catheter variations including but not limited to: a single extrusion catheter with dual lumens, and a coaxial catheter with an inner lumen being for guidewire and outer lumen being tubular with a slit, gap or other opening. In a coaxial catheter the deployment line would be positioned between the inner tubular guidewire lumen extrusion and the outer tubular extrusion which incorporates a gap or slit in its wall.

Additional lumens can be added to the catheter if desired. Additional lumens could be used to allow inflation of a balloon, a second deployment line, or for other functions. Likewise, variations including an expandable medical device or other components are also contemplated by the present invention.

Unlike other conventional sheath pull back systems which use a full length coaxial tube arrangement, the outside diameter proximal end of the catheter of the present invention catheter remains completely stationary during deployment. This lack of movement during deployment makes it easier for a physician to precisely locate a stent or other device. A yet further improvement over conventional sheath pull back systems is that, a coaxial cover (228) may be positioned over the slit in the dual lumen catheter proximal of the deployment line/sheath transition. The coaxial cover is designed to slide proximally with the sheath movement. Additionally, the deployment line can be located closer to the center of inertia of the catheter, thus minimizing the tendency of the extrusion to bow during deployment line tensioning. A section of this flexible or rigid coaxial cover can be designed to keep the slit closed proximal of the deployment line/sheath transition ensuring the deployment line remains within the catheter. The coaxial cover (228) may be comprised of elastic materials or may be formed of a material which wrinkles, pleats or otherwise compresses during sheath pull back prior to deployment. Additionally, the proximal end of this cover may or may not be attached to the catheter extrusion. If it is not attached, since the proximal end can translate relative to the catheter extrusion during device deployment, the cover may comprise a rigid material. Suitable materials for the cover include elastomers, thermoplastics, thermosets (e.g. polyimide) fluoropolymers such as polytetrafluoroethylene further including porous expanded polytetrafluoroethylene. The proximal end of the cover is preferably at least the length of the device(s) being deployed and relatively thin with a wall thickness less that 0,508 mm (0.020 inches). It is more preferred that the wall thickness be less than 0,127 mm (0.005 inches) and or most preferred that the wall thickness be less than 0,0254 mm (0.001 inches).

Figure 22A illustrates a retracted sheath pull back catheter of the present invention with a longitudinal slit post-retraction of the sheath. As shown, upon retraction of the sheath, the proximal end of the coaxial cover (proximal of sheath/deployment line junction) is compressed to maintain the positioning of the deployment line in the catheter while permitting longitudinal movement of the sheath in relation to the axis of the catheter. Figure 22B shows a cross section of the present invention post deployment. As is seen, the deployment line lumen (223) is vacant at locations distal of the sheath/deployment line junction.

Although particular embodiments of the present invention have been shown and described, modifications may be made to the deployment system and assembly without departing from the spirit and scope of the present invention.

### EXAMPLES

### Example 1

This example describes the construction of a deployment system of the present invention. Construction of the system began with the preparation of a distal catheter shaft for receiving an expandable stent. Once the distal catheter was prepared, the expandable stent was placed within a sheath - deployment line. The distal catheter portion of this combination was attached to a primary catheter shaft. The deployment line portion was then routed through the primary catheter to a control knob. The control knob was part of a hub located proximally on the primary catheter. The sheath portion of the sheath - deployment line was in the form of a single-walled tube.

A tubular material three inches long was obtained from Burnham Polymeric, Inc., Glens Falls, NY for use as the distal catheter shaft. The tube was made of a polyether block amide material, commonly known as PEBAX® resin and reinforced with a stainless steel braid. The outer diameter (OD) was 1.01mm and the inner diameter (ID) was 0.76mm. An endoprosthesis mounting member in the form of a compressible material was then placed on the catheter.

To place the endoprosthesis mounting member on the catheter, the catheter was mounted on a mandrel having an outer diameter of 0.74mm. A film of porous expanded polytetrafluoroethylene (ePTFE).was obtained according to the teachings in U.S. Patent No. 5,814,405, issued to Branca, which is incorporated herein by reference. A discontinuous coating of fluorinated ethylene propylene (FEP) was applied to one side of the ePTFE material in accordance with U.S. Patent No. 6,159,565 issued to Campbell.et al. An edge of the ePTFE - FEP composite film two inches wide was attached with heat to the catheter shaft. After initial attachment, the film was wrapped around the catheter shaft forty-five (45) times under light tension. With every fifth wrap of the flm, and on the final layer, the film is further attached to itself with heat supplied by a soldering iron.

This procedure provided a endoprosthesis mounting member in the form of a compressible material, or compliant "pillow," on the distal catheter shaft. The expandable stent was mounted over the endoprosthesis mounting member. The endoprosthesis mounting member provides a means of retaining an expandable stent on the catheter shaft during storage, delivery to an implantation site, and deployment of the expandable stent at the implantation site. Optionally, the endoprosthesis mounting member may be reinforced with a thin coating of an elastomeric material such as silicone, urethane, and/or a fluoroelastomer.

An eight (8) cell, 6mm diameter, nitinol stent was obtained from Medinol Ltd., Tel-Aviv, Israel. The stent was placed over the endoprosthesis mounting member of the catheter in an expanded state. The combination was placed within a machine having a mechanical iris that compacts or compresses the stent portion of the assembly onto the endoprosthesis mounting member. While retained in the mechanical iris machine, the stent was reduced in temperature from room temperature (c. 22°C) to approximately five degrees centigrade (5°C). At the reduced temperature, the iris machine was actuated to compact, or collapse, the stent onto the endoprosthesis mounting member. While in the refrigerated and compressed configuration, the catheter, endoprosthesis mounting member, and stent were placed within a sheath-deployment line of the present invention.

The sheath - deployment line having a length equal to, or greater than, the length of the final deployment system was made as follows. A length of stainless steel mandrel (c. 1m), measuring 1.89mm in diameter was covered with a tubular extruded ePTFE material having an overall length of about 200cm. The tubular ePTFE material had an outer diameter of 1.41mm, a wall thickness of 0.05mm, and an average longitudinal tensile strength of 3.52kgf with an average circumferential strength of 0.169kgf. The tubular ePTFE material also had an average mass/length of 0,1552 g/cm (0.0473 (0.0473g/ft) with an average Matrix Tensile Strength of 4,70368 Atm (69,125 PSI). At one end (proximal end), the tubular ePTFE material was bunched together on the mandrel, while the opposite end (distal end) of the ePTFE material remained smooth on the mandrel.

The first few centimeters of the tubular ePTFE material was sacrificed and the next 5cm of the distal end (smoothed end) of the extruded ePTFE material was then reinforced with a composite fluoropolymer material as follows. The ePTFE-covered mandrel was attached to retaining chucks on a film-wrapping machine. A first reference line located approximately 5cm from the end of the smooth portion of the extruded ePTFE material was circumferentially drawn around the material with a permanent marker (SHARPIE^{®}). A 5cm wide composite membrane made of expanded polytetrafluoroethylene (ePTFE) and fluorinated ethylene propylene. (FEP) was applied proximal from the first reference line on the extruded ePTFE material so the FEP portion of the composite membrane was against the extruded ePTFE material. The composite membrane was wrapped around the ePTFE covered mandrel two times so that the primary strength of the extruded ePTFE material was oriented perpendicular to the longitudinal axis of the mandrel. The composite membrane was initially tacked in place on the extruded ePTFE material with heat applied from a soldering iron. The composite ePTFE/FEP material had a density of about 2.14g/cm³, a thickness of 0.005mm, and tensile strengths of about 340 KPa (about 49,000 psi) in a first direction and of about 120 KPa (about 17,000 psi) in a second direction (perpendicular to the first direction). The tensile measurements were performed on an Instron Tensile Machine (Instron Corporation, Canton, MA) at 200mm/min. load rate with 2.5cm (one inch) jaw spacing.

Material of the sheath - deployment line construction adjacent to the reinforced portion was smoothed out along the mandrel and a second reference line was drawn around the material 5cm from the first reference line.

A second portion of the sheath - deployment line construction was reinforced as follows. A second reference line was drawn around the extruded ePTFE material 5cm from the proximal end of the first reinforced portion. Using the second reference line to align a 2cm wide strip of the abode-mentioned ePTFE/FEP composite membrane, the composite membrane was wrapped once around the remaining portion of the extruded ePTFE material to form a second reinforced portion of the sheath - deployment line of the present invention. The second reinforced portion was about 2cm in length. The composite reinforcing membrane material was attached to the extruded ePTFE material as described above, with the exception that the major strength component of the material was parallel to the axis of the mandrel.

Any air trapped in the construction Was removed by applying a sacrificial layer of ePTFE tightly around the construction. A one inch (2.54cm) wide film of ePTFE was helically overwrapped around the reinforced portion of the construction. Two layers of the ePTFE film were applied in one direction and two layers were applied in the opposite direction. The construction with sacrificial layers were then placed in an oven heated to 320°C for eight minutes. Upon removal from the heated oven, the combination was allowed to cool to room temperature. The sacrificial ePTFE material was then removed.

The construction was then removed from the mandrel and another mandrel (1.83mm diameter X 30.5cm long) inserted into the reinforced end of the construction. With the mandrel supporting the reinforced end, a 5mm long slit was made proximal to the reinforced portion of the sheath - deployment line construction. A second mandrel was placed inside the construction up to the 5mm slit where it exited the construction. The proximal portion of the sheath - deployment line construction was converted into a filament by placing the proximal end into the chucks of the film wrapper chucks and rotating the film wrapper approximately 2,800 times white the mandrel with the reinforced construction was immobilized. After the construction was spun into a filament, the filament was strengthened by briefly applying heat to the filament with a soldering iron set at 450°C. The strengthened filament was smoothed and rendered more uniform in diameter by passing the filament over a 1.8cm diameter X 3.8cm long dowel heated to approximately 320°C. The filament was passed over the heated dowel at a 45° angle under slight tension. This process was repeated two more times over the entire length of the filament.

The filament portion of the sheath - deployment line of the present invention was routed through a lumen of a primary catheter and connected to a control knob. The control knob was part of a hub located at the proximal end of the primary catheter. When the deployment line portion of the sheath - deployment line was pulled, the sheath portion was retracted from around the stent.

### Example 2

This example describes the construction of a deployment system of the present invention. Construction of the system begins with the preparation of a distal catheter shaft for receiving an expandable stent. Once the distal catheter was prepared, the expandable stent was placed within a sheath - deployment line. The distal catheter portion of this combination was attached to a primary catheter shaft. The deployment line portion was then routed through the primary catheter to a control knob. The control knob was part of a hub located proximally on the primary catheter. The sheath portion of the sheath - deployment line was in the form of a double-walled tube.

A tubular material three inches long was obtained from Burnham Polymeric, Inc., Glens Falls, NY for use as the distal catheter shaft. The tube was made of a polyether block amide material, commonly known as PEBAX® resin and reinforced with a stainless steel braid. The outer diameter (OD) was 1.01 mm and the inner diameter (ID) was 0.76 mm. A endoprosthesis mounting member in the form of a compressible material was then placed on the catheter. To place the endoprosthesis mounting member on the catheter, the catheter was mounted on a mandrel having an outer diameter of 0.74 mm. A film of porous expanded polytetrafluoroethylene (ePTFE) was obtained according to the teachings in U.S. Patent No. 5,814,405, issued to Branca. A discontinuous coating of fluorinated ethylene propylene (FEP) was applied to one side of the ePTFE material in accordance with U.S. Patent No. 6,159,565, issued to Campbell et al. An edge of the ePTFE - FEP composite film two inches wide was attached with heat to the catheter shaft. After initial attachment, the film was wrapped around the catheter shaft forty-five (45) times under light tension. With every fifth wrap of the film, and on the final layer, the film is further attached to itself with heat. This procedure provides a endoprosthesis mounting member on the distal catheter shaft. The expendable stent is mounted over the endoprosthesis mounting member. The endoprosthesis mounting member provides a means of retaining an expandable stent on the catheter shaft during storage, delivery to an implantation site, and deployment of the expandable stent at the implantation site. Optionally, the endoprosthesis mounting member may be reinforced with a thin coating of an elastomeric material such as silicone, urethane, and/or a fluoroelastomer.

An eight (8) cell, 6mm diameter, nitinol stent was obtained from Medinol Ltd., Tel-Aviv, Israel. The stent was placed over the endoprosthesis mounting member of the catheter in an expanded state. The combination was placed within a machine having a mechanical iris that compacts or compresses the stent portion of the assembly onto the endoprosthesis mounting member. While retained in the mechanical iris machine, the stent was reduced in temperature from room temperature to approximately five degrees centigrade (5°C). At the reduced temperature, the iris machine was actuated to compact, or collapse, the stent onto the endoprosthesis mounting member. While in the refrigerated, compressed configuration, the catheter, endoprosthesis mounting member, and stent were placed within a sheath - deployment line of the present invention.

The sheath - deployment line having a length equal to, or greater than, the length of the final deployment system was made as follows. A stainless steel mandrel measuring 1.73 mm in diameter was covered with a sacrificial layer of ePTFE. The sacrificial ePTFE material aids in removal of the sheath - deployment line from the mandrel. Two wraps of a thin, polytetrafluoroethylene (PTFE) membrane were applied to the mandrel. The ePTFE membrane was applied so the primary strength of the film was oriented parallel with the longitudinal axis of the mandrel. The film was initially tacked in place with heat applied with a soldering iron. The membrane thickness measured about 0.0002" (0.005 mm) and had tensile strengths of about 49,000 psi (about 340 KPa) in a first direction and of about 17,000 psi (about 120 KPa) in a second direction (perpendicular to the first direction): The tensile measurements were performed at 200mm/min. load rate with a 1" (2.5 cm) jaw spacing. The membrane had a density of about 2.14g/cm³. The membrane was further modified by the application of an FEP coating on one side in accordance with U.S. Patent No. 6,159,565, issued to Campbell et al. Next, two wraps of another ePTFE film made according to the teachings of Bacino in U.S. Patent No. 5,476,589 and further modified with a discontinuous layer of an FEP material applied to one side of the ePTFE film were applied to one end of the construction (approx. 2,54 cm (1" wide)) U.S. Patent No. 5,476,589. These two wraps had the primary strength direction of the film oriented perpendicular to the mandrel's longitudinal axis. These layers of film provide additional "hoop" or "radial" strength to the sheath - deployment line construct. The mandrel and sheath - deployment line construct were placed in an air convection oven obtained from The Grieve Corporation, Round Lake, IL, and subjected to a thermal treatment of 320°C for 12 minutes. After air cooling, the ePTFE/FEP tube construct was removed from the mandrel and the sacrificial ePTFE layer removed. In this example, a length of sheath - deployment line extending beyond the end of the stent was provided. The additional length of sheath - deployment line was folded back over sheath portion enclosing the stent to form a double-walled construct. The double-walled sheath - deployment line had an inner wall and an outer wall. The inner wall was against the stent and the outer wall included the integral deployment line portion of the construct. The construct was then attached to a primary catheter shaft using heat and standard materials.

The deployment line portion of the sheath - deployment line was made by splitting the sheath - deployment line along its length from a proximal end up to, but not including, the sheath portion enclosing the stent. The material thus obtained was gathered into a filament by rolling the material. Heat was applied to the material to set the material in the filamentous form. The deployment line filament was routed through a lumen in the primary catheter and connected to a control knob. The control knob was part of a hub located at the proximal end of the primary catheter. When the deployment line portion of the sheath - deployment line was pulled, the sheath portion was retracted from around the stent.

### Example 3

This example describes the incorporation of a means for initiating or maintaining conversion of the sheath portion of the sheath - deployment line to deployment line by introducing perforations and intentional stress risers into the sheath.

The sheath - deployment line from Example 2 is modified as follows. Prior to rolling the sheath portion into a double-walled construct and loading the stent therein, the sheath is perforated and/or supplied with "stress risers" that facilitate in separation of the tubular sheath upon retraction of the deployment line portion. An appropriate laser for making the perforations or stress risers is a 20 watt CO₂ laser obtained from Universal Laser Systems, Scottsdale, AZ. To form the perforations in the sheath portion, the sheath is placed on a sandblasted stainless steel mandrel and exposed to the laser to cut a series of holes in a part of the tube that will subsequently serve as the outer wall of the double-walled construct. The geometry of the holes can be varied depending on the application. The perforated sheath portion is used on a deployment line system of the present invention as described in Example 2. In this example, tension applied to the deployment line portion at the hub end of the catheter results in retraction of the sheath from around the stent and also results in parting the sheath at the perforations. As the sheath portion is separated, the sheath material becomes convertible to deployment line.

### Example 4.

This example describes the incorporation of a means for initiating or maintaining conversion of the sheath portion of the sheath - deployment line to deployment line by the use of an appropriate splitting means.

The primary catheter from Example 2 is modified as follows. The primary portion of the catheter is provided with a notch in the wall in 180 degrees opposition and slightly distal to the entry point of the deployment line portion into the catheter lumen. The notch is further modified to provide a small cutting edge in the notch. In one embodiment, the cutting edge is simply attached to the notch with heat, adhesives, and the like. In another embodiment, the cutting edge is formed by exposing a portion of a metallic braid used to reinforce the catheter shaft and forming the braid into a cutting edge. In this example, tension applied to the deployment line portion at the hub end of the catheter results in retraction of the sheath from around the stent and also results in parting the sheath at the perforations. As the sheath portion is separated, the sheath material becomes convertible to deployment line.

### Example 5

This example describes the construction of a deployment system of the present invention for use in the delivery and deployment of both self-expanding as well as balloon expandable devices. The deployment system of this example utilizes an endoprosthesis mounting member in the form of an inflatable balloon.

A sheath - deployment line having a length equal to, or greater than, the length of the final deployment system is made as follows. A stainless steel mandrel measuring 1.73 mm in diameter is covered with a sacrificial tube of ePTFE. The sacrificial ePTFE material aids in removal of the sheath - deployment line from the mandrel. Two wraps of a thin, polytetrafluoroethylene (EPTFE) membrane is applied to the mandrel. The ePTFE membrane is applied so the primary strength of the film is oriented parallel with the longitudinal axis of the mandrel. The film is initially tacked in place with heat applied with a soldering iron. The membrane thickness measured about 0.0002" (0.005 mm) and had tensile strengths of about 49,000 psi (about 340 KPa) in a first direction and about 17,000 psi (about 120 KPa) in a second direction (perpendicular to the first direction). The tensile measurements are performed at 200mm/min. load rate with a 1 inch (2.5 cm) jaw spacing. The membrane has a density of about 2.14g/cm³. The membrane is further modified by the application of a fluorinated ethylene propylene (FEP) coating on one side in accordance with U.S. Patent No, 6,159,565, issued to Campbell et al Next, two wraps of another ePTFE film made according to the teachings of Bacino in U.S. Patent No. 5,476,589, and further modified with a discontinuous layer of an FEP material applied to one side of the ePTFE film are applied to one end of the construction (approx. 1" wide). These two wraps have the primary strength direction of the film oriented perpendicular to the mandrel's longitudinal axis. These layers of film provide additional "hoop" or "radial" strength to the sheath - deployment line construct. The mandrel and sheath - deployment line construct are placed in an air convection oven obtained from The Grieve Corporation, Round Lake, IL, and subjected to a thermal treatment of 320°C for 12 minuted. After air cooling, the ePTFE/FEP tube construct is removed from the mandrel and the sacrificial ePTFE layer removed. Placement of this construct over an expandable stent and formation of a deployment line portion wherefrom is described below.

As seen in Figure 10, a balloon expandable NIRflex™ stent (14), available from Medinol Ltd, Tel-Aviv, Israel, is placed over and compacted around a deflated and collapsed angioplasty balloon mounted on a delivery catheter shaft (19). The angioplasty balloon is made in accordance with US 5,752,934 to Campbell et al. and available from W.L. Gore & Associates, Inc., Flagstaff, AZ under the tradename APTERA® angioplasty balloon. The APTERA® angioplasty balloon serves as an endoprosthesis mounting member (18a) for receiving arid retaining the compacted stent (14).

While the stent is confined in a compacted configuration, a length of sheath - deployment line (12) is placed over the compacted stent and extended beyond the end of the stent. The additional length of sheath - deployment line is folded back over sheath portion enclosing the stent to form a double-walled construction. The double-walled sheath - deployment line has an inner wall and an outer wall. The inner wall is against the stent and the outer wall includes the integral deployment line portion of the construct.

The deployment line portion of the sheath - deployment line is made by splitting the sheath - deployment line along its length from the proximal end toward the distal end for a distance. The slit can range in length from about one centimeter to substantially the entire length of the sheath - deployment line construction up to, but not including, the sheath portion enclosing the stent. It is preferred to form the deployment line portion near the proximal end of the delivery catheter. The material thus obtained is gathered into a filament by rolling the material. Heat is applied to the material to set the material in the filamentous form. The sheath - deployment line is routed through a dedicated lumen in the delivery catheter and exits at a hub where the deployment line portion is attached to a control knob. The control knob is part of a hub located at the proximal end of the primary catheter. When tension is applied to the deployment line portion of the sheath - deployment line, the sheath portion retracts from around the stent. Removal of the sheath portion from the underlying stent frees the stent to expand. The NIRflex™ stent of this example is expanded by inflating the APTERA® angioplasty balloon. Once the stent is expanded, the balloon is deflated and the delivery catheter along with the sheath - deployment line construction removed from the implant recipient. When self-expanding stents are used in the present invention, the balloon is useful as an endoprosthesis mounting member.

### Example 6

This example describes the construction of a deployment system of the present invention utilizing a deployment assembly of the present invention.

A deployment system according to any of the above-examples can be used with a deployment assembly of the present invention. For purposes of illustration, this example will be described with reference to the deployment system described in Example 5 having an endoprosthesis mounting member in the form of an inflatable balloon.

As seen in Figures 14 - 17, a deployment assembly of the present invention (100) is configured to be connected to a delivery catheter (101) so a lumen (111) of the delivery catheter is in fluid communication (32) with the inflatable endoprosthesis mounting member described in Example 5 and a first pressurizable chamber (109) of the deployment assembly (100).

In this example, the first pressurizable chamber (109) in the form of a plastic syringe was fitted with a rubber plunger (102) attached to a means for moving the plunger in the chamber (104, 106) to generate, maintain, or reduce pressure in the deployment system. Means for actuating the deployment line portion were provided in the form of second pressurizable chamber (124) in fluid communication with the first pressurizable chamber (109). The second pressurizable chamber in the form of a plastic syringe was provided with a moveable rubber piston (122) placed therein and attached to the deployment line portion through a plastic connecting rod (120).

As plunger (102) was moved into the first pressurizable chamber (109), fluid pressure increased in the chamber (102), the catheter lumen (111), and the endoprosthesis mounting member causing the endoprosthesis mounting member to exert radial force against the overlying balloon expandable NIRflex™ stent (14). Simultaneously, fluid pressure in the second pressurizable chamber (124) increased and began to move piston (122) actuating the attached deployment line (114) and retracting the sheath from the balloon expandable NIRflex™ stent (14).

### Example 7

In this example, a deployment system of the present invention is further fitted with a pressure-storing apparatus. As shown in Figures 18 - 20, various pressure-storing apparatuses can be affixed to the deployment system in fluid communication with an endoprosthesis mounting member.

The pressure-storing apparatus (130) of this example is made by attaching a plastic syringe to the deployment system having a diaphragm (132) in the form of a movable rubber plunger in the syringe. The diaphragm (132) defines a first airtight chamber (139) containing compressible gas (136) and a second chamber (137) in fluid communication with a lumen of an inflatable endoprosthesis mounting member (18a). The compressible gas (136) in the first chamber (139) is increased in pressure as pressure is increased in the pressurizable chamber (109) and the endoprosthesis mounting member (18a). Once application of pressure in the pressurizable chamber (109) is stopped, the compressed gas (136) in the first airtight chamber (139) presses against the diaphragm (132) and exerts pressure on fluid in the deployment system to maintain or increase fluid pressure in the endoprosthesis mounting member (18a).

## Claims

1. A deployment system (200) for an endoluminal device comprising:
a. a catheter (19) having a length, an outer wall and.at least one lumen (223); a longitudinal opening (229) in said outer wall that accesses said at least one lumen (223);
b. a retractable continuous thin-walled sheath (12) at least partially enclosing the catheter (19); and
c. a deployment line (16) configured to pull with the retractable sheath (12);
wherein said deployment line (16) is integral with the sheath (12) extending through said longitudinal opening (229), and into said lumen (223), so that rotation of the catheter (19) relative to the deployment line (16) is prevented.

2. The deployment system (200) of claim 1, further comprising means on said catheter (19) for initiating conversion of said retractable continuous thin-walled sheath (12) to deployment line (16).

3. The deployment system (200) of claim 1, wherein said retractable continuous thin-walled sheath (12) is in the form of a polymeric film.

4. The deployment system (200) of claim 3, wherein said polymeric film is in a tubular form having at least one wall.

5. The deployment system (200) of claim 3, wherein said polymeric film is in a tubular form having two or more walls.

6. The deployment system (200) of claim 1, wherein said deployment line (16) comprises a fluoropolymer.

7. The deployment system (200) of claim 5, wherein said fluoropolymer comprises polytetrafluoroethylene.

8. The deployment system (200) of claim 7, wherein said polytetrafluoroethylene is porous expanded polytetrafluoroethylene.

9. The deployment system (200) of claim 3, wherein said retractable sheath (12) comprises a fluoropolymer.

10. The deployment system (200) of claim 9, wherein said fluoropolymer comprises polytetrafluoroethylene.

11. The deployment system (200) of claim 10, wherein said polytetrafluoroethylene is porous expanded polytetrafluoroethylene.

12. The deployment system (200) of claim 1, wherein the sheath (12) is not attached to said catheter (19).

13. The deployment system (200) of claim 10, wherein at least a portion of said PTFE has a density greater than 2.1 g/cc.

14. The deployment system (200) of claim 10, wherein at least a portion of said PTFE has a density greater than 2.0 g/cc.

15. The deployment system (200) of claim 10, wherein at least a portion of said PTFE has a density greater than 1.5 g/cc.

## Patentansprüche

1. Entfaltungssystem (200) für ein endoluminales Gerät, aufweisend:
a. einen Katheter (19) mit einer Länge, einer Außenwand und mindestens einem Lumen (223); eine längliche Öffnung (229) in der Außenwand, die Zugang zu dem mindestens einen Lumen (223) hat;
b. eine einziehbare durchgängige dünnwandige Umhüllung (12), die den Katheter (19) zumindest teilweise umschließt; und
c. eine Entfaltungsschnur (16), die konfiguriert ist, um mit der einziehbaren Umhüllung (12) gezogen zu werden;
wobei die Entfaltungsschnur (16) mit der Umhüllung (12) einstückig ist und sich durch die längliche Öffnung (229) und in das Lumen (223) erstreckt, sodass eine Drehung des Katheters (19) relativ zu der Entfaltungsschnur (16) verhindert wird.

2. Entfaltungssystem (200) nach Anspruch 1, ferner aufweisend Mittel auf dem Katheter (19) zum Einleiten einer Umkehr der einziehbaren durchgängigen dünnwandigen Umhüllung (12) zu der Entfaltungsschnur (16).

3. Entfaltungssystem (200) nach Anspruch 1, wobei die einziehbare durchgängige dünnwandige Umhüllung (12) in Form einer Polymerfolie vorliegt.

4. Entfaltungssystem (200) nach Anspruch 3, wobei die Polymerfolie in einer schlauchartigen Form mit mindestens einer Wand vorliegt.

5. Entfaltungssystem (200) nach Anspruch 3, wobei die Polymerfolie in einer schlauchartigen Form mit mindestens zwei oder mehr Wänden vorliegt.

6. Entfaltungssystem (200) nach Anspruch 1, wobei die Entfaltungsschnur (16) ein Fluorpolymer aufweist.

7. Entfaltungssystem (200) nach Anspruch 5, wobei das Fluorpolymer Polytetrafluorethylen aufweist.

8. Entfaltungssystem (200) nach Anspruch 7, wobei das Polytetrafluorethylen ein poröses expandiertes Polytetrafluorethylen ist.

9. Entfaltungssystem (200) nach Anspruch 3, wobei die einziehbare Umhüllung (12) ein Fluorpolymer aufweist.

10. Entfaltungssystem (200) nach Anspruch 9, wobei das Fluorpolymer Polytetrafluorethylen aufweist.

11. Entfaltungssystem (200) nach Anspruch 10, wobei das Polytetrafluorethylen ein poröses expandiertes Polytetrafluorethylen ist.

12. Entfaltungssystem (200) nach Anspruch 1, wobei die Umhüllung (12) nicht an dem Katheter (19) befestigt ist.

13. Entfaltungssystem (200) nach Anspruch 10, wobei mindestens ein Teil des PTFE eine höhere Dichte als 2,1g/cm³ hat.

14. Entfaltungssystem (200) nach Anspruch 10, wobei mindestens ein Teil des PTFE eine höhere Dichte als 2,0 g/cm³ hat.

15. Entfaltungssystem (200) nach Anspruch 10, wobei mindestens ein Teil des PTFE eine höhere Dichte als 1,5 g/cm³ hat.

## Revendications

1. Système de déploiement (200) pour dispositif endoluminal comprenant :
a. un cathéter (19) ayant une longueur, une paroi externe et au moins une lumière (223), un orifice longitudinal (229) dans ladite paroi externe qui permet l'accès à ladite au moins une lumière (223) ;
b. une gaine continue rétractable à paroi mince (12) entourant au moins en partie le cathéter (19) ; et
c. une ligne de déploiement (16) conçue pour tirer avec la gaine rétractable (12) ;
ladite ligne de déploiement (16) étant incorporée à la gaine (12) en s'étendant à travers ledit orifice longitudinal (229), et dans ladite lumière (223), de façon à empêcher la rotation du cathéter (19) par rapport à la ligne de déploiement (16).

2. Système de déploiement (200) selon la revendication 1, comprenant en outre des moyens sur ledit cathéter (19) pour initier la conversion de ladite gaine continue rétractable à paroi mince (12) en ligne de déploiement (16).

3. Système de déploiement (200) selon la revendication 1, ladite gaine continue rétractable à paroi mince (12) étant sous la forme d'un film polymère.

4. Système de déploiement (200) selon la revendication 3, ledit film polymère étant sous une forme tubulaire comportant au moins une paroi.

5. Système de déploiement (200) selon la revendication 3, ledit film polymère étant sous une forme tubulaire comportant deux parois ou plus.

6. Système de déploiement (200) selon la revendication 1, ladite ligne de déploiement (16) comportant un fluoropolymère.

7. Système de déploiement (200) selon la revendication 5, ledit fluoropolymère comportant du polytétrafluoroéthylène.

8. Système de déploiement (200) selon la revendication 7, ledit polytétrafluoroéthylène étant du polytétrafluoroéthylène expansé poreux.

9. Système de déploiement (200) selon la revendication 3, ladite gaine rétractable (12) comportant un fluoropolymère.

10. Système de déploiement (200) selon la revendication 9, ledit fluoropolymère comportant du polytétrafluoroéthylène.

11. Système de déploiement (200) selon la revendication 10, ledit polytétrafluoroéthylène étant du polytétrafluoroéthylène expansé poreux.

12. Système de déploiement (200) selon la revendication 1, ladite gaine (12) n'étant pas fixée audit cathéter (19).

13. Système de déploiement (200) selon la revendication 10, au moins une partie dudit PTFE ayant une masse volumique supérieure à 2,1 g/cc.

14. Système de déploiement (200) selon la revendication 10, au moins une partie dudit PTFE ayant une masse volumique supérieure à 2,0 g/cc.

15. Système de déploiement (200) selon la revendication 10, au moins une partie dudit PTFE ayant une masse volumique supérieure à 1,5 g/cc.
